**Europäisches Patentamt**

**European Patent Office**

**Office européen des brevets**

(19)

(11) Publication number : **0 491 638 A2**

# EUROPEAN PATENT APPLICATION

(12)

(21) Application number : **91460051.5**

(22) Date of filing : **06.12.91**

(51) Int. Cl.$^5$ : **C07C 227/10,** C07C 229/26,
B01J 2/12, B01J 2/16

(30) Priority : **17.12.90 US 628648**
**24.10.91 US 778291**

(43) Date of publication of application :
**24.06.92 Bulletin 92/26**

(84) Designated Contracting States :
**AT BE CH DE DK ES FR GB GR IT LI LU NL SE**

(71) Applicant : **ARCHER DANIELS MIDLAND COMPANY**
**4666 Faires Parkway**
**Decatur, Illinois 62525 (US)**

(72) Inventor : **Verhoff, Francis H.**
**951 Stevens Creek Circle**
**Decatur, Illinois 62526 (US)**

(74) Representative : **Le Guen, Louis François**
**CABINET Louis LE GUEN 38, rue Levavasseur**
**B.P. 91**
**F-35802 Dinard Cédex (FR)**

(54) **Method of making granulated L-lysine.**

(57) A dust free, free-flowing, L-LysineHCl granular product is made from a liquid solution or slurry by a spray granulation process. The L-LysineHCl is sprayed onto an agitated bed of L-LysineHCl seed particles until the size of the particles reach a harvest size. In one embodiment, the agitated bed is a fluidized bed. In other embodiments, the agitated bed is a rotating mechanical device including either a drying drum or a disk and cone combination, where the particles are grown in an intermittent spray/dry cycle. Then the harvested particles are sorted for size. Those particles which are a non-acceptable oversize are ground to seed size and returned to the agitated bed. Those particles which are a non-acceptable undersize are remelted, purified, and returned to the liquid solution. Other salts of L-Lysine may also be used, such as an L-Lysine sulfate salt, an L-Lysine phosphate salt, and an L-Lysine calcium salt.

EP 0 491 638 A2

This is a continuation-in-part of Serial No. 07/628,648 filed December 17, 1990.

This invention relates to a process for producing granulated L-LysineHCl (L-Lysine monohydrochloride) by removing water from a solution containing L-LysineHCl, and more particularly, to an accomplishment of such a water removal by using a spray granulation technique in order to produce a better appearing dust free, solid end product which is easier to handle. Usually, the L-LysineHCl produced in this manner is used as a feed additive for animals.

For many years, an L-LysineHCl solid has been produced by a process of fermentation, purification, crystallization and drying. After fermentation, the L-Lysine has been recovered from the fermentation broth by an ion exchange, which produces a liquid which is substantially a L-Lysine free base. This solution is then concentrated by evaporation.

Hydrochloric acid is added to the concentrated L-Lysine free base in order to form L-LysineHCl. This concentrated L-LysineHCl solution is crystallized to produce a product in the form of L-LysineHCl dihydrate (L-LysineHCl:$2H_2O$). This crystallized solid is thereafter dried to have less than one percent moisture.

Unfortunately, this conventional product may have a considerable number of shortcomings. For example, it is dusty. During a handling of the product, the dust results in a loss of valuable material and sometimes causes an incomplete formulation. Also, human working conditions are made less healthful and more difficult by the dust contributed by the L-LysineHCl. Sometimes, the conventional product is not as free flowing as would be desired, thus causing additional material handling problems. Sometimes it is difficult to break up lumps which are formed at the time of end use when the granules are re-liquefied.

Accordingly, an object of this invention is to provide new and improved means for and methods of producing a solid, dust free, granular L-LysineHCl.

Another object is to obtain dried L-Lysine granules of acceptable size which are free-flowing, non-dusting, non-friable and which do not have the above described drawbacks in usage.

In keeping with an aspect of this invention, these and other objects are accomplished by spray granulating L-LysineHCl in a warm fluidized bed. The fluidized bed is originally seeded with solid L-LysineHCl particles obtained from any suitable source. Then, liquid L-LysineHCl is sprayed over the particles while they are simultaneously dried in the fluidized bed to increase the size of the seeds until they reach an acceptable range. The resulting particles are then sorted for size and bagged for sale. The particles having non-acceptable sizes either are too small or are ground to become smaller than an acceptable size and returned to the fluidized bed as the seeds for the next generation of products. The inventive process may be carried out in either discrete batches or a continuous process.

According to another aspect of the invention, various mechanical agitation devices may be used instead of a conventional fluidized bed. For example, one device is an agitated drying drum granulator which features an upwardly directed stream of warm drying air originating at approximately an axis of a rotating drum. In the lower part of the drum (i.e. below the source of drying air), seed particles are wetted either by a spray or by a pool of liquid. The rotating drum has internal fins which carry the wetted seed particles up to the stream of drying air where they are tumbled and somewhat dried before falling back into the bottom of the drum for another wetting/drying cycle. Hence, this system uses an alternating wet/dry cycle as distinguished from conventional fluidized beds where the spraying and drying steps are carried out simultaneously.

Another mechanical agitating device includes two coaxial chambers separated by an annular space, the outer chamber being a substantially closed area. A rotating disk is located at the bottom of the inner chamber, a gap being formed between a periphery of said disk and an inner one of said coaxial chambers. Seed particles are rolled on the surface of the disk responsive to the rotation thereof. As it is so rolled, the seed particles are sprayed in order to make them grow in size. Hence, this equipment tends to produce a better shaped particle as distinguished from a randomly shaped particle which grows in a fluidized bed where the growing particle may jut out in any direction. A stream of warm and drying air blows the wet particles falling through said gap back up through the annular space and onto the spinning disk where the particles are dried. The particles fall back where they are again wetted. The air also prevents the particles from falling through the crack between the disk and the inner chamber wall.

Two embodiments of mechanical agitation devices for drying particles are shown in the attached drawings, in which:

Fig. 1 is a partly broken away perspective view showing a mechanical agitator in the form of drying drum granulator;

Fig. 2 is a cross-section (taken along line 2-2) of the mechanical agitator of Fig. 1;

Fig. 3 is a schematic diagram of a mechanical agitator in the form of co-axial chambers having a rotary disk device; and

Fig. 4 schematically shows a system which may use any drying device.

A standard process which has been used in the prior art for making solid L-LysineHCl particles might be described somewhat, as follows:

(a) Ferment a broth in which a microorganism grows and produces L-Lysine.

(b) Pass the broth through a screen or membrane with suitably sized pores in order to remove solids which may form in the broth.

(c) Pass the resulting fluid through an ion exchanger which separates the L-Lysine from the broth to provide a free-based L-Lysine which contains some ammonia.

(d) Place the output of the ion exchanger in an evaporation-stripper system which removes the ammonia for producing a more pure free-based L-Lysine preferably of higher concentration.

(e) Add hydrochloric acid which produces L-LysineHCl.

(f) Crystallize the L-LysineHCl, which forms a L-LysineHCl dihydrate (L-LysineHCl:2H$_2$O).

(g) Dry the crystallized L-LysineHCl:2H$_2$O to remove the dihydrate water, leaving a dry particle which may be bagged and sold.

The end product of this conventional process (i.e. the bagged and sold product resulting from step (g)), has a number of problems and short comings, such as, dusting, non-free flowing, and friable particles. Sometimes the end product gives a generally unfavorable presentation of the L-Lysine.

According to the inventive process, the seeds are initially taken from a suitable standard commercially available L-LysineHCl material (e.g. the bagged product of step (g)). The particles of step (g) are dissolved and re-liquefied in water or a dilute L-Lysine solution to form the liquid L-LysineHCl which is more pure than that of step (e) because the crystallization further refines the product. An alternative is to begin the inventive steps with a product which is provided earlier in the above described standard process. For example, start with the L-LysineHCl dihydrate of step (f) and eliminate at least the drying step (g). The liquid after the addition of HCl (step (e)) is another convenient starting point. Consequently, intermediate steps, such as those associated with crystallization, may be eliminated.

In addition to the monohydrochloride salt of L-Lysine, there are other salts which may be processed through the spray granulation process. Among these salts are sulfate, phosphate, and calcium salts. These salts would be especially desirable if sulfur, phosphorus, or calcium is needed in the diet of the animals being fed the lysine supplement.

The specific salts are formed in step (e) above by adding the desired acid or base instead of the hydrochloric acid. For example to form the L-Lysine sulfate salt, sulfuric acid would be added. For the L-Lysine phosphate salt, phosphoric acid is added. For the calcium lysinate, calcium hydroxide is added. Regardless of what is added, the resultant liquid formed in step (e) is sprayed on the bed formed of suitable particles. According to experimental tests, these salts can be processed as described herein with varying production rates and temperature constraints, thus forming the desired granulated product.

In at least one test, the inventive process used a fluidized bed device which was manufactured by the Vector Company of Marrion, Iowa, and which is sold under the trademark "Flow Coater". Tests were actually run in "Flow Coater" fluidized bed devices with either a 500-pound or a 10-pound capacity bowl. Another test used a fluidized bed device (capacity ~ 2kg) which was manufactured by Aeromatic Inc. of Columbia, MD. Devices of these types are also available from other sources.

The feed to the fluidized bed is via a spray nozzle in the dryer (usually located at a height which is somewhat near the upper surface of the stationary bed in the dryer). Various nozzles can be used to supply the gas, but those supplied by the Schlick Company having an address at Coburg, Germany appeared to work well in the following examples.

The fluidized bed device has a bed which may be partially filled with L-Lysine salt particles. For example, a dry L-LysineHCl powder may be obtained by using L-LysineHCl which has been produced by almost any of the known state of the art processes. This dry powder may be separated according to particle size into a range of less than about 1000 micrometers (18 mesh). Another useful partiCle size is in the range of about 150 to 600 micrometers.

After the initial cycle and once the process becomes self-sustaining, the dry powder for the fluidized bed comes from recycling material back into the dryer by using either the particles which are too small or the particles which are too large and have been ground to a smaller size.

A suitable gas (air, for example) is blown through the bed of particles to expand and fluidize it. The fluidizing and drying air is supplied to the dryer at a temperature between approximately 70 and 240°C, and preferably between 110 and 190°C. This air heats the dry L-LysineHCl powder to the desired temperature of about 50 to 140°C, and preferably between 75 and 100°C.

While the bed is fluidized, an L-Lysine salt solution (e.g. L-Lysine monohydrochloride) is simultaneously sprayed onto the particles and dried in order to increase their size, after which time, they are removed, sorted and bagged. The sprayed fluid is composed of about 20 to 85 percent L-LysineHCl and at a temperature between substantially 30 to 100°C. Higher concentrations of L-LysineHCl are possible only at higher temperatures; however, it is not essential that all of the L-LysineHCl be dissolved since a slurry can be handled by the inventive process.

This sprayed fluid passes through a nozzle (either a one or two phase nozzle may be used) onto the drying fluidized bed. For single fluid nozzles, the pressures required to fluidize the bed are between about 15 and 25 bars. For the two fluid nozzles (air and L-LysineHCl solution), the air pressure should be between about 0.5 and 10 bars.

Initially, a number of tests were run making a spray granulated L-LysineHCl. These tests began by purchasing L-LysineHCl particles from a number of different commercial suppliers or liquid was taken from step (e) of the conventional process, i.e. the liquid was taken from the output from HCl addition tank. For each test which began with particles, they were first dissolved in water to make an L-LysineHCl solution which was sprayed onto the particles in the fluidized bed of seeds. The spray is continued until there is a desired increase in bed weight which is usually achieved when the bed weight is increased by a factor in the range of substantially two to ten. Preferably the increase is from three to six.

Operating the fluidized bed according to this invention yields the following particle size fractions.

1. About 1 to 50 percent of the particles are less than 40 mesh (250 micrometers).

2. About 15 to 85 percent of the particles are between 18 mesh (1000 micrometers) and 40 mesh (250 micrometers).

3. About 5 to 70 percent of the particles are greater than 18 mesh (1000 micrometers).

The inventive process for spray granulation of an L-Lysine salt solution or specifically for an L-LysineHCl solution produced from crystallized L-LysineHCl or HCl neutralized L-Lysine free base proceeds in a manner such that:

a) L-LysineHCl in the form of a dry powder preferably with most of the powder in the size range of 30 to 200 mesh (75 to 600 micrometers) is placed in a fluidized bed drying device (spray granulator) with the fluid bed maintained at about 30 to 140°C or preferably in the range of about 40 to 100°C.

b) After the condition described in step a), a solution is added by spraying it in an atomized form onto the fluidized bed at a rate which increases the size of the particles in the bed.

c) The L-LysineHCl particles are removed from the fluidized bed after a suitable time depending upon the desired particle size, and are separated into groups according to the particle size fractions by any suitable and known size filtering means.

d) The resultant spray granulated L-LysineHCl has particle sizes which range between approximately 18 and 40 mesh (425 to 1000 micrometers), which are discharged for bagging as the end product.

e) The particles which are outside of the acceptable range of sizes (either larger or smaller) are ground to a size which is smaller than the largest acceptable end product and are returned to the fluidized bed drying device.

This process can be carried out in either a discontinuous or a continuous manner. The continuous process is preferred.

## Example 1

A fluidized bed drying device was filled with 2.0 kg of powdered L-LysineHCl, all particles of which passed through 60 mesh (250 micrometers). The inlet air temperature of 79°C was supplied to the dryer at 75 cfm (2.1 cubic meters/min) to heat the powder until the outlet air temperature reached 60°C. At that time, the spray was initiated at 90 ml/min of a water solution containing 60 percent L-LysineHCl (prepared by dissolving anhydrous L-LysineHCl feed grade in water).

The spray was continued for 38 minutes with different atomizing air pressures (between 1 and 3 kg/sq.cm. or 14 and 42 psi). The outlet air temperature dropped gradually to 55°C during the 38 minutes while the spraying continued. Approximately 60 percent of the resulting particles or granules were in the size range of 18 to 40 mesh (425 to 1000 micrometers). The remainder of the particles were smaller than this size.

## Example 2

A fluidized bed drying device was filled with 44.5 kg of previously spray granulated L-LysineHCl which was ground so that most of it is particles or granules in the 30 to 60 mesh range (250 to 600 micrometers). The air flow was set at approximately 2100 cfm with an inlet air temperature of about 126°C. When the outlet air temperature reached 80°C, the spray (a three headed Schlick nozzle) was started at approximately 2.2 liters/min with a concentration of about 65% L-LysineHCl (made by dissolving anhydrous feed grade L-LysineHCl in water).

The spray was continued for about 80 minutes with an atomizing air pressure fluctuating between approximately 58 and 80 psi. The outlet air temperature varied between about 83 and 93°C. The resultant particles were of a size such that about 40% of the particles were greater than 18 mesh (1000 micrometers) and 60% of the particles were between 18 mesh and 40 mesh (425 to 1000 micrometers). There was a trace of material passing through 40 mesh.

## Example 3

A fluidized bed drying device was filled with 45.5 kg of previously spray granulated L-LysineHCl having a particle size greater than 18 mesh (1000 micrometers) which was ground so that most of it is in the 30 to 60 mesh range (250 to 600 micrometers) (1% greater than 30 mesh and 4% less than 60 mesh). The air flow was approximately 1200 cfm (34 cubic meters/min) with an inlet air temperature of about 124°C.

When the outlet air temperature reached substantially 100°C, the spray (a three headed Schlick nozzle) was started at 1.4 liters/min with a concentration of 60% L-LysineHCl (L-LysineHCl feed grade dissolved in water). The spray was continued for 41 minutes with an atomizing air pressure of approximately 65 psi. The outlet air temperature started at 100°C and gradually decreased to 71°C over the duration of the spray. The resultant particles were mostly in the 18 to 40 mesh range with about 20% being larger. There were some lumps which were caused by a plugged nozzle.

## Example 4

A fluidized bed drying device was filled with 45.5 kg of previously spray granulated L-LysineHCl, which was oversized material ground so that most of it was in about the 30 to 60 mesh range (250 to 600 micrometers). The air flow was approximately 1700 cfm (48 cubic meters/min) with an inlet temperature of substantially 122°C. When the outlet air temperature reached approximately 80°C, the spray (a three headed Schlick nozzle) was started at approximately 1.7 1/min with a concentration of approximately 60% L-LysineHCl. This solution was formed by adding HCl to an L-Lysine free base solution which had been ion exchanged from a fermentation broth until a pH of approximately 6.0 was achieved.

The spray was continued for about 58 minutes with an atomizing air pressure of approximately 75 psi. The outlet air temperature decreased from substantially 80°C to about 62°C during the drying. Most of the resulting particles were within the desired range of 18 mesh to 40 mesh (425 to 1000 micrometers), with about 20 percent of the particles being greater than 18 mesh (1000 micrometers).

## Example 5

A fluidized spray bed device was filled with 750 gms of lysine salt solid with a size range from 200 to 600 mm. A solution was made by dissolving 1.12 kg of L-Lysine free base and 0.3808 kg of 98% sulfuric acid into 1.5 kg of water. The air flow rate was set at approximately 70 cfm, varying between 50 in the beginning and ending near 100 cfm with an inlet air temperature of approximately 80°C. When the outlet air temperature reached approximately 40°C, the spray of L-Lysine sulfate solution was started at a rate of 12.5 gm/min and raised in increments to 43 gm/min.

The spray was continued for about 1 hour and 50 minutes until the solution was essentially utilized. The atomizing air pressure in the two fluid nozzle was initially set at 1.0 bar and was raised to 1.5 bar, approximately halfway through the experiment. The outlet air temperature remained in the range of approximately 42 to 48°C during the run. The resultant particles had a size range from 300 to 1400 mm.

## Example 6

A fluidized spray bed device was filled with 750 gms of lysine salt solid with a size range from 200 to 600 mm. The L-Lysine phosphate solution was made by dissolving 0.897 kg of L-Lysine free base and 0.685 kg of 88% phosphoric acid into 1.5 kg of water. The air flow rate was set at approximately 125 cfm varying between 120 in the beginning and ending near 130 cfm with an initial inlet air temperature of approximately 80°C which was stepped up to 90°C to avoid formation of large lumps in the fluidized bed. When the outlet air temperature reached approximately 40°C, the spray of L-Lysine phosphate solution was started at a rate of 12.5 gm/min. Higher liquid feed rates were tried but a lumping of the bed prevented the sustaining of these higher rates. Therefore, the solution feed rate was maintained at the 12.5 gm/min rate.

The spray was continued for about 1 hour and 17 minutes until the bed became too lumpy to continue. The atomizing air pressure in the two fluid nozzle was maintained at approximately 1.5 bar throughout the experiment. The outlet air temperature remained in the range of approximately 70°C during the run. The resultant particles had a size range from 250 to 1100 mm.

### Example 7

A fluidized spray bed device was filled with 750 gms of lysine salt solid with a size range from 200 to 600 mm. The calcium L-Lysinate slurry was made by adding 1.318 kg of L-Lysine free base and 0.336 kg of calcium hydroxide into 1.5 kg of water. The air flow rate was set at approximately 110 cfm varying between 70 in the beginning and ending near 125 cfm with an initial inlet air temperature of approximately 80°C which was stepped up to 90°C shortly after the spray was started into the fluidized bed. When the outlet air temperature reached approximately 50°C, the spray of calcium L-lysinate slurry was started at a rate of 12.0 gm/min.

The spray was continued for about 1 hour and 15 minutes with constant interruptions because the nozzle would plug. The atomizing air pressure in the two fluid nozzle was maintained at approximately 1.5 bar throughout the experiment. The outlet air temperature remained in the range between 58 and 70°C during the run. The resultant particles had a size range from 200 to 800 mm.

Surprisingly, it has been discovered that spray granulation of L-LysineHCl (spraying into a fluidized bed of drying solids) produces free-flowing, non-dusting, non-friable granulates, when fermentation produced and crystallized L-LysineHCl is redissolved in water and spray granulated in a specific manner and without binders. Further, it has also been discovered that the ion exchanged, purified, evaporation concentrated, and HCl neutralized L-LysineHCl solution can be formed into the spray granulates without the intermediate step of crystallization and without requiring binders.

These discoveries are particularly surprising because friable, dusty granulates would normally be expected since it is known that L-LysineHCl crystallizes from a solution, as the dihydrate. As it dries, an anhydrous material forms on the surfaces of the granulates in the bed. Normally, it would be thought that this material should give rise to friable granulates and dust. Or stated another way, it had been thought that the spraying of particles in a fluidized bed would result in layers of the dihydrate forming on the surfaces of the particles. These layers would break off as the water was removed and cause more, not less, dust. The surprise was that such dust particles did not so form.

An advantage of this invention is that the process includes a suitable arrangement for making it possible to recover a specific particle size fraction as the end product and to provide dry solids for maintaining the dry product in the fluid bed.

Fluidized beds are, in general, well known devices. They usually involve a container having a bed of particles which are lofted by a stream of air or other pressurized fluid. As the particles are so lofted, the behavior of the bed changes from that of a solid to that of a fluid. When the air is heated, it also dries the particles.

The embodiment of the invention which has been described thus far contemplates a use of a spray head in the middle of a bed of fluidized seed particles which simultaneously wets and dries the seed particles. The seed particles are sprayed while they are being dried by a draft of warm fluidizing air. Therefore, the sprayed liquid clings to the seed particle while it is drying and forming a particle coating which increases the diameter of the seed particle.

In addition to systems in which the L-LysineHCl solution is continuously wetted and dried in a fluidized bed of particle seeds, other systems may use mechanically agitated beds in which the L-LysineHCl solution is sprayed or otherwise put onto a bed (stationary, moving or tumbling) of seed particles with no air other than the ambient atmosphere. Later the wetted particles are dried in a stream of drying air. For convenience of expression, all of these beds (fluidized and mechanically agitated) are generically called "agitated beds" herein.

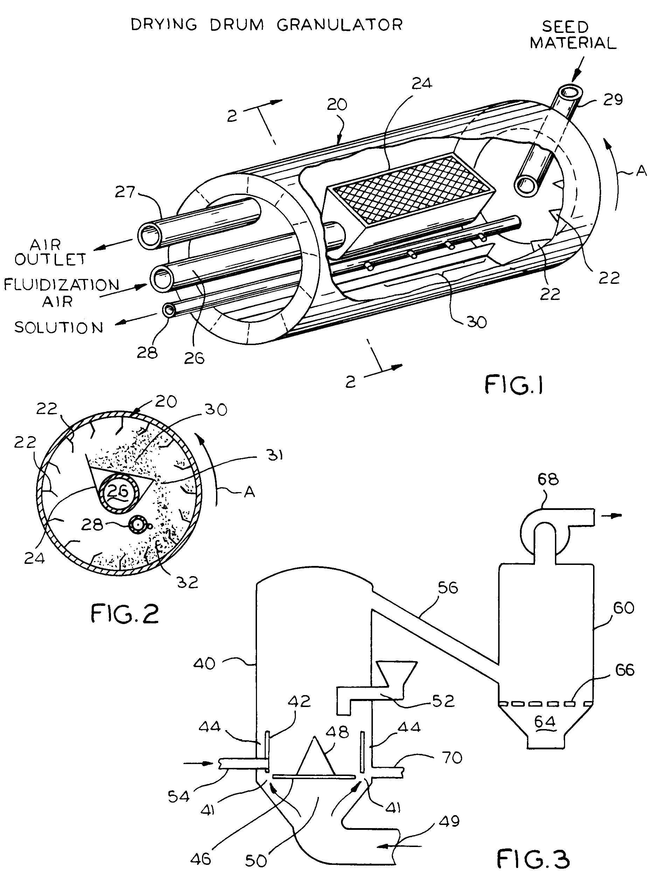

One such non-fluidized, mechanically agitated bed device for forming particles with a wet/dry cycle is seen in Figs. 1 and 2. Here, a revolving drum 20 contains a plurality of inwardly directed fins or flights 22 extending longitudinally along an inside cylindrical surface of the drum. A stationary tray or trough 24 having an open top is suspended more or less along an axis of the drum. A first pipe 26 is used to force drying air into and out of the top of the trough 24. A second exhaust pipe 27 draws the heated air from the drum. A third pipe 28 leading to a manifold 30 which sprays a solution into the drum. Seed particles are dumped into the drum 20, via a fourth pipe 29.

The operation is best illustrated by Fig. 2. The drum 20 is rotating in direction A. The seed particles dumped through pipe 29 are picked up by the internal fins or flights 22 and carried to the top of the drum 20 at 30 where they fall under the force of gravity onto the trough 24. The drying air blowing through pipe 26 and out of the top of the trough 24 creates a turbulence in the air which agitates and lofts the seed particles in the general area immediately above the trough. The particles in this turbulent air are dried, soon escape and fall out and back into the bottom of the drum, as shown at 31, recirculating on the fins 22 to return to the turbulent drying air, at 30, above the trough.

The solution enters pipe 28 and is sprayed at 32 onto the tumbling particles. This spray wets the particles so that the seed particles grow in a wet/dry cycle as they are sprayed, dried, and returned to the bottom of the drum, as the particles cascade at 30 and 31.

Fig. 3 shows another device having a mechanically agitated bed for increasing the size of seed particles. An outer chamber 40 has an inner wall 42 which together form a separate and co-axial chamber having an air passage way through annular space 44 between the walls of the two co-axial chambers. A rotating disk 46 with a cone 48 centrally formed thereon is positioned to rotate within the inner chamber wall 42. There is a circular gap and screen between the periphery of the rotating disk 46 and the inner wall 44. The surface of cone 48 may be either smooth or textured. Heated drying air 49 is driven into a plenum 50 and on into the annular space 44 between the periphery of disk 46 and inner chamber wall 42. The heat of this air warms and dries the wetted seed particles. The driving force of the air causes the particles falling through the gaps between the disk 46 and the bottom wall 42 to circulate through annular space 44 and over the top of the wall 42, falling back onto the disk, as shown by arrows A-D in Fig. 3. The screen 41 prevents any particles from reaching plenum 50.

Seed particles are introduced into the inner chamber 42 and onto the disk 46 via a suitable passageway 52. A liquid spray-may be introduced through one or more pipes 54 extending into the inner chamber and onto the seed particles above disk 46, as they roll over. In Fig. 3, it is possible to spray a fluid onto some of the seed material and to dry other of the particles at the same time. A suitable passageway 56 enables the very fine particles which escape and move with the drying air, to pass into a dust recovery chamber 60. The air fan 68 draws the heated drying air. The separation plate 66 allows the dust particles to fall into area 64 for collection and recycling. After the particles grow large enough by the alternately spraying and drying cycle, the particles are withdrawn via a suitable pipe 70 and are sent to a bagger.

When particles are to be formed in Fig. 3, the initial seeds are loaded into the inner processing chamber 42, rolled by the rotation of the disk 46 and cone 48, wetted by the spray through pipe 54. The mechanically agitated bed of forming particles are set into a rolling and circular motion by the rotating disk 46 and cone 48 which tends to promote a greater degree of geometrical uniformity of shape as the particles dry. The heated airflow through space 44 dries and conveys the particles over the wall 42 and back into the inner chamber (inside wall 42) for further wetting by the spray. The dust particles are carried out pipe 70 and into a bag filter. Thus, the particles are sequentially sprayed with liquid while inside wall 42 and dried while inside the annular space 44 between walls 40, 42. Many different parameters (such as the spray rate, seed feeding, temperature, etc.) are variable in order to optimize the process. The shape and size of the particles during the forming stage are influenced by the speed and surface structure of the rotary disk. Therefore, the action of this (Fig. 3) device is different from that of a conventional fluidized bed.

The mechanical agitator types of structure shown in Figs. 1 and 2 usually cause an intermittent cycle of wetting and drying the seed particle, as distinguished from the conventional fluidized bed where the wetting and drying are simultaneous and continuous. Therefore, to test the feasibility of alternately wetting and drying, a conventional fluidized bed, where spraying is normally continuous, was operated in a stop/start manner. The fluidizing air was turned off and the seed particles were sprayed for measured periods of time. Then, the spray was turned off and the warm fluidizing air was introduced to dry the sprayed particles. The spray/dry cycle was repeated in a number of different ways in order to simulate the action of the device of Figs. 1 and 2.

**Example 8**

An unfluidized spray bed device was filled with 2.0 kg of L-LysineHCl granules of the size range of 300-1000mm. The L-LysineHCl solution (50% L-Lysine at 50°C) was sprayed onto the granules at a rate of 20 gr/min air while the bed of granules was stationary and no fluidizing, drying air was being directed through the bed. The spraying flow of the L-LysineHCl solution was stopped. Thereafter, the drying air (80°C) was introduced into the fluidized bed at the rate of 140 cfm. The intermittent and sequential spraying and drying were performed successfully for several wet/dry cycles, as follows:

| NUMBER OF WET/DRY CYCLES | SPRAY TIME MIN. | DRY TIME MIN. |
|---|---|---|
| 10 | 12 | 18 |
| 4 | 15 | 15 |

7

| 6 | 20 | 10 |
| 5 | 30 | 30 |
| 5 | 40 | 20 |
| 8 | 50 | 10 |

After each WET/DRY cycle, the particles were inspected. There was a successful granulation. No indication of agglomerating was found. From these tests, it was concluded that although the action is different from that of a conventional fluidized bed, the drum type dryer (Figs. 1, 2) produced satisfactory L-LysineHCl particles.

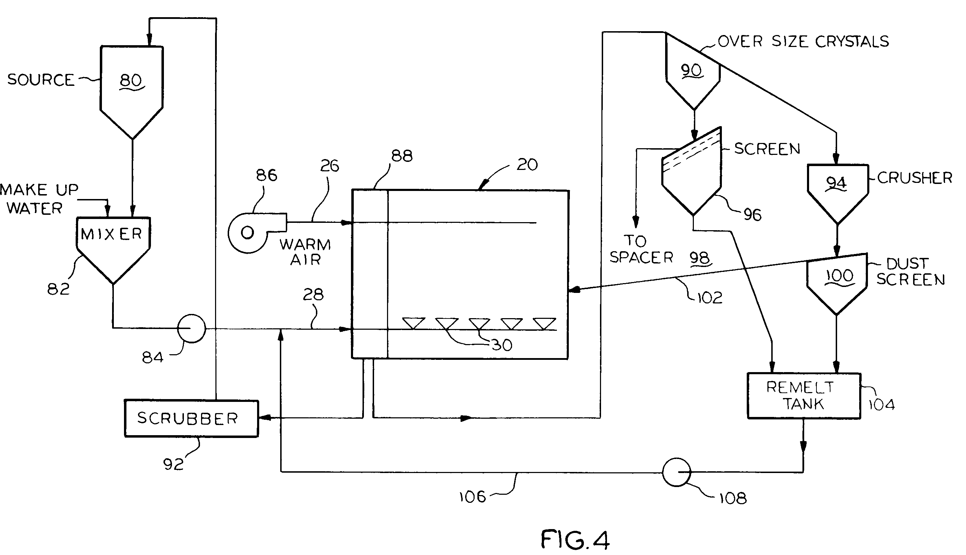

Fig. 4 illustrates a system which might be used with any of the drying devices discussed herein, although this particular system is shown as using the drying drum granulator 20 of Figs. 1, 2.

A liquid source of the L-LysineHCl material is shown at 80. Any suitable amount of make up water (or other liquid) is added to the liquid particle material at a mixer 82. A pump 84 drives the fluid through pipe 28 and the manifold 30 of spray heads. A blower 86 drives warm air through pipe 26 and into drum 20.

The particles are grown in drum 20 to the desired size in the manner shown and explained in connection with Fig. 2. Then a suitable separator 88 separates the grown particles and forwards them to an oversize separator 90. Separator 88 also sends any fine powder which may emerge with the particles to a scrubber 92 which removes any foreign matter and then returns it to the powder source 80.

The grown particles which reach oversize separator 90 are screened for size. Any oversize particles are sent to crusher 94 where they are reduced in size. The remainder of the particles reaching and passing through oversize separator 90 are forwarded to screener 96. Those particles which do not pass through screener 96 are sent to a bagger 98 for sale or consumption.

The particles which are crushed at 94 are separated at dust screen 100 into a size suitable for use as seed particles which are returned to the drying drum 20 via path 102 in order to start the next generation of particles. Smaller crushings are a dust of particles that are too small and are forwarded to a remelt tank 104. The remelted material is returned at 106 to the drying drum 20 under the urging of a pump 108.

The disk type dryer of Fig. 3 may operate in a similar manner.

Those who are skilled in the art will readily perceive how to modify the invention. Therefore, the appended claims are to be construed to cover all equivalent structures which fall within the true scope and spirit of the invention.

## Claims

1. A process for producing solid spray granulated L-LysineHCl in the form of flowable, non-dusting, non-friable particles, said process comprising'the steps of:
   providing an agitated bed of L-LysineHCl particles in a drying device,
   spraying an L-LysineHCl solution onto said agitated heated bed of L-LysineHCl particles, and
   drying the sprayed particles in heated air stream, the sprayed L-LysineHCl being substantially free of noticeable amounts of binders.

2. The process of claim 1 wherein the dried L-LysineHCl particle size is between substantially 425 and 1000 micrometers.

3. Thy process of claim 1 wherein the dried L-LysineHCl particle size range is between substantially 100 and 2500 micrometers.

4. The process of claim 1 and the added step of grinding the sieved L-LysineHCl particles which are larger than a predetermined size, said grinding reducing larger particles to become a smaller size which may serve as seed for said agitated bed, and returning the ground smaller sized L-LysineHCl particles to the agitated bed as seeds for the next generation of L-Lysine particles.

5. The process according to claim 1, wherein said agitated bed includes seeds of L-LysineHCl-in the form of a dry powder in the size range of approximately 30 to 200 mesh (75 to 600 micrometers), said dry powder being placed in said bed of a drying device and maintained within a temperature range of approximately 30 to 110°C substantially throughout said spraying step.

6. The process of claim 5 wherein the maintained temperature is in the range of approximately 40 to 100°C.

7. The process according to claim 1 wherein said agitated bed is a fluidized bed of L-LysineHCl particles, and the added step of adding said L-LysineHCl solution onto the fluidized bed which increases the size of said particles in the fluidized bed.

8. The process of claim 1 wherein said agitated bed includes a horizontally oriented rotating drum, a plurality of fins on an inside of said drum for carrying said particles from a bottom to a top of said drum, said process comprising the further steps of wetting said particles in said bottom of said drum with said L-LysineHCl solution, and drying said particles while in an agitated bed at said top of said drum, said drying step including a directing of an upward stream of heated air for lofting said wetted particles while they are being dried.

9. The process of claim 1 wherein said agitated bed includes a circular wall with a rotating surface formed by at least a horizontally oriented disk, a circular gap formed between a periphery of said disk and said circular wall, said particles rolling over said surface to form an agitated bed of particles and the added steps of spraying L-LysineHCl on said particles while they are in a surface of said disk, and heating said particles while they are blown from said gap up an outside surface of said wall and over the top of said wall to fall back toward said disk.

10. The process according to any one of the claims 7-9 and the added steps of removing the L-LysineHCl particles of increased size from the agitated bed of L-LysineHCl particles after a suitable period of time which is adequate to produce a desired final end-product particle size, and sieving said agitated bed of L-LysineHCl particles according to their size.

11. The process according to claim 1, further comprising:
(a) a continuous drying process including the steps of maintaining said agitated bed of L-LysineHCl particles at between about 40 and 110°C;
(b) spraying a solution of L-LysineHCl onto the L-LysineHCl particles and drying these particles with a warm air stream;
(c) removing L-LysineHCl particles from the agitated bed;
(d) separating the removed particles-into fractions of desired size; and
(e) recycling the particles of non-desired size to the agitated bed in order to maintain the continuous process.

12. The process of claim 11 and the further step wherein said steps (a), (b) are performed simultaneously in a fluidized bed.

13. The process of claim 11 and the further step wherein said steps (a), (b) are performed sequentially in a mechanically agitated bed.

14. The process of claim 11 wherein said recycling step (e) comprises the further step of grinding oversize particles to form smaller sized particles.

15. A process for manufacturing L-LysineHCl particles comprising the steps of:
(a) fermenting L-Lysine in a broth medium;
(b) removing at least some elements including L-Lysine from the fermentation broth medium of step (a);
(c) passing the elements removed from said fermentation in step (b) into an ion exchanger for separating said L-Lysine from said broth medium and producing a purer L-Lysine solution;
(d) concentrating this L-Lysine solution;
(e) adding hydrochloric acid to said L-Lysine separated in step (d) to make L-LysineHCl;
(f) spraying said L-LysineHCl of step (e) onto an agitated drying bed of L-LysineHCl particles, said spraying being at a rate which increases the size of said particles in said drying bed; and
(g) recovering said increased size particles after a said increased size reaches a predetermined size.

16. The process of claim 15 and the added steps of:
(d1) crystallizing said L-LysineHCl of step (e); and

9

(d2) dissolving said crystallized L-LysineHCl in water to provide a liquid which is sprayed in step (f).

17. The process of claim 15 and the added steps of:
crystallizing said L-LysineHCl of step (e); and
dissolving said crystallized L-LysineHCl in a dilute L-LysineHCl solution to provide a liquid which is sprayed in
step (f).

18. The process of claim 15 wherein said agitated bed of particles have sizes which are initially in the range of about 30-200 mesh (75-600 micrometers) and said bed is held at a temperature substantially in the range of about 30-110°C.

19. The process of claim 15 wherein said agitated bed of particles have sizes which are initially in the range of about 30-200 mesh (75-600 micrometers) and said bed is held at a temperature substantially in the range of about 40-100°C.

20. The process of claim 15 wherein step (f) comprises the added step of atomizing said L-LysineHCl as it is sprayed onto
said agitated bed.

21. The process of claim 15 wherein step (f) is continued until said increased particle size is within the range of about 100-2500 micrometers.

22. The process of claim 15 wherein step (f) is continued until said increased size is within the range of about 425-1000 micrometers.

23. The process of claim 15 wherein said agitated bed is a continuously fluidized bed drying process includes the steps of maintaining the bed of L-LysineHCl particles at between about 40 and 110°C;
continuously spraying a solution of L-LysineHCl onto the fluidized bed;
continuously removing part of the fluidized bed material;
separating the removed particles into fractions of desired size as end-product; and
recycling the particles of a non-desired size to the fluidized bed in order to maintain the process.

24. The process of claim 15 wherein said agitated bed is a rotating mechanical device and said spraying step of step (f) involves spraying said L-LysineHCl onto particles while they are being rotated in said device, and the step of spraying and drying being carried out in a two step wet/dry cycle.

25. The process of claim 15 wherein said agitated bed is a rotating mechanical device and step (f) involves simultaneously spraying and drying said particles.

26. A process for manufacturing L-LysineHCl particles comprising the steps of:
(a) fermenting L-Lysine in a broth medium;
(b) removing at least some elements of said broth medium including L-Lysine from the fermentation of step (a);
(c) passing the elements removed from said fermentation in step (b) into an ion exchanger for separating said L-Lysine from said broth;
(d) passing the L-Lysine separated in step (c) through an evaporator to remove ammonia therefrom in order to produce freebase L-Lysine;
(e) adding hydrochloric acid to said L-Lysine freebase in order to make L-LysineHCl;
(f) atomizing and spraying said L-LysineHCl onto an agitated and drying bed of L-LysineHCl particles maintained at a temperature substantially in the range of about 30-110°C, said spraying being at a rate which increases the size of said particles in said bed until they reach a size in the range of about 100-2500 micrometers: and
(g) recovering and sorting said particles of increased size after a said increased size reaches said range.

27. The process of claim 26 and the added step of concentrating the L-Lysine in the solution of step (d).

28. The process of claim 26 wherein the L-LysineHCl particles which are larger than a predetermined size are ground to a smaller size suitable for seeding said agitated drying bed to provide seeds for the next generation of L-Lysine particles.

29. A process according to claim 26, wherein said agitated bed is a fluidized bed, said process further comprising:

a continuously fluidized bed drying process including the steps of maintaining the bed of L-LysineHCl particles at between about 40 and 110°C;

continuously spraying a solution of L-LysineHCl onto the fluidized bed;

continuously removing part of the fluidized bed material;

separating the removed particles into desired size fractions as end-product; and

recycling the particles, at least some of which are a non-desired size, to the fluidized bed in order to maintain the process.

30. A process for producing a solid spray granulated salt of L-Lysine in the form of a flowable, non-dusting, non-friable, particles, said process comprising the steps of:

heating an agitated bed of salts of L-Lysine particles in a drying device, and

spraying a salt of L-Lysine solution onto said heated bed of dry L-Lysine particles, and

drying the solution onto the bed, the sprayed salt of L-Lysine solution being substantially free of noticeable amounts of binders.

31. The process of claim 30 wherein said salt of L-Lysine is L-Lysine sulfate salt.

32. The process of claim 30 wherein said salt of L-Lysine is L-Lysine phosphate salt.

33. The process of claim 30 wherein said salt of L-Lysine is L-Lysine calcium salt.

34. A product made by the process of any one of the claims 1, 15, 26 or 30.

DRYING DRUM GRANULATOR

SEED MATERIAL

FIG.1

FIG.2

FIG.3

FIG.4